# EUROPEAN PATENT SPECIFICATION

(11) **EP 0 571 670 B1**
(45) Date of publication and mention of the grant of the patent: **05.02.1997**
(21) Application number: 92304711.2
(22) Date of filing: 26.05.1992
(51) Int. Cl.: A61K 31/125, A61K 31/35

(54) **Pharmaceutical compositions for use as mast cell stabilizer**
Pharmazeutische Zusammensetzungen zur Verwendung als Mastzellstabilisator
Compositions pharmaceutiques employés comme stabilisateur des granulocytes basophyles

(43) Date of publication of application: 01.12.1993
(60) Divisional application: 95104176.3
(73) Proprietor: THE PROCTER & GAMBLE COMPANY, Cincinnati, Ohio 45202 (US)
(72) Inventor: Bazin, Ann Louise, London W11 3AH (GB); Koochaki, Patricia Elaine, Cincinnati, Ohio 45242 (US)
(74) Representative: Brooks, Maxim Courtney

(56) References cited:
- DE-A- 1 807 747
- TAKEDA KENKYUSHOHO, vol. 40, nos. 1-2, 1981, pages 27-36; S. NAGASHIMA et al.: "Isolation of substances with anti-histamine like activity from "Shin-i""
- PATENT ABSTRACTS OF JAPAN, vol. 16, no. 190 (C-937), 8th May 1992; & JP-A-4 026 619 (LOTTE CO., LTD) 29-01-1992
- PATENT ABSTRACTS OF JAPAN, vol. 16, no. 183 (C-936), 6th May 1992; & JP-A-4 023 967 (LOTTE CO., LTD) 28-01-1992
- Z. ALLG. MED., vol. 55, no. 9, 1979, pages 599-603, Hippokrates Verlag GmbH, Stuttgart, DE; A. BACKHAUS et al.: "Schnupfenspray Larylin"
- CLINICAL ALLERGY, vol. 10 (suppl), 1980, pages 481-489, Blackwell Scientific Publications; R.E.C. ALTOUNYAN: "Review of clinical activity and mode of action of sodium cromoglycate"
- JOURNAL OF TOXICOLOGY AND ENVIRONMENTAL HEALTH, vol. 18, 1986, pages 91-101, Hemisphere Publishing Corp.; L.G. COCKERHAM et al.: "Disodium cromoglycate, a mast-cell stabilizer, alters postradiation regional cerebral blood flow in primates"
- RD-319097

## Description

This invention relates to the use of a bicyclic aromatic flavour compound for the manufacture of a composition for use as a mast cell stabilizer.

Approximately 10 % of the population of the United Kingdom and the United States suffer from seasonal allergic rhinitis (hay fever). The symptoms of hay fever are nasal, ocular and palatial irritation, sneezing and hypersecretion following exposure to allergens. In Europe the main allergens are grass and tree pollens, hence allergic rhinitis commonly occurs during the spring and summer months. The symptoms of hay fever are believed to be due to the stimulation of H-1 receptors by histamine, followed by reflex activation of parasympathetic nerves causing further increases in nasal secretion and obstruction. Histamine is released in the tissues by the antigen-antibody reaction. Conventional antihistamines are histamine H-1 receptor antagonists. They diminish or abolish the main actions of histamine on the body. They effect this by a mechanism that involves the occupation of receptor sites in the effector cells to the exclusion of histamine. They do not prevent the production or release of histamine.

Most of the tissue histamine is present in mast cells. When antigen comes into contact with the tissues, some of the mast cells are disrupted and subsequently histamine is released from them into the surrounding tissues. Other substances such as prostaglandins, leukotrienes and other inflammatory intermediaries are also released. These, in turn, cause cascade mechanisms which lead to inflammation and symptoms of the allergic response. The nervous system is also stimulated by some of these intermediaries to cause vasodilatation and oedema. Acid sulphate-containing polysaccharides are also present in the large granules of the mast cells. Histamine is stored in the granules as an ionic complex with a basic protein, which itself binds strongly to the acid polysaccharide, leaving a weak negative charge, such that the histamine can be easily displaced under the influence of appropriate metal cations.

Aromatic flavouring agents such as menthol, camphor and eucalyptol have been widely used in pharmaceutical decongestant compositions for the treatment of the symptoms of coughs and colds. Menthol is the active component in peppermint oil and it has been shown to offer significant symptomatic improvement in patients with irritable bowel syndrome (Proceedings of the BPS, 10-12 April, 1985, 293P). It is believed that menthol produces inhibition of gastrointestinal smooth muscle by decreasing the influx of extra-cellular calcium through potential-dependent channels while having no effect on the intracellular mobilization of calcium. But there has been no indication in the art that any of the common flavouring compounds are useful *per se* in stabilizing mast cells or regulating histamine activity or that they are valuable for the treatment of allergic rhinitis and related diseases.

Sodium cromoglycate has been used as a prophylactic in the treatment of asthma and allergic rhinitis since the early 1970's. The precise mechanism by which the sodium cromoglycate works is still not clear but it has been suggested that it acts by preventing the release of histamines. GB-A-1,144,906 discloses the preparation of sodium cromoglycate.

Many clinical studies in humans conducted during the last 20 to 25 years have consistently demonstrated that sodium cromoglycate is both safe and effective in reducing the severity of symptoms associated with seasonal allergic rhinitis.

RD-319097 discloses solutions of sodium cromoglycate incorporating an aromatic flavouring agent. Flavouring agents can be selected from menthol, eucalyptol, camphor and methyl salicate, the concentration said to be typically from 0.001 % to 0.5 %. The concentration of sodium cromoglycate is in the range from 0.1 % to 10 %.

Takeda Kenkyushoho, 40(1/2), 1981, 27-36 discloses the antihistamine activity of camphor.

Z.Allgemeinmed., 55(9), 1979, 599-603 discloses the use of Larylin (a medicament containing camphor and eucalyptol) for treating acute rhinitis.

DE-A-1807747 discloses the use of camphor in the treatment of asthma.

However there is no mention whatsoever in these documents of the use of camphor or eucalyptol for the stabilization of mast cells.

Accordingly, it is an object of the invention to provide pharmaceutical compositions having improved effectiveness in the treatment of allergic rhinitis and as mast cell stabilizers.

### Summary of the Invention

The present invention relates to the use of a bicyclic aromatic flavour compound for the manufacture of a composition for the stabilization of mast cells wherein the bicyclic aromatic flavour compound is selected from camphor, eucalyptol and mixtures thereof.

The pharmaceutical compositions herein are preferably provided in the form of an isotropic aqueous solution, especially preferred embodiments additionally comprising a solubilizing agent for solubilizing the bicyclic aromatic flavour compound. The composition can be provided in the form of an aerosol spray.

All levels and ratios herein are given by weight of total composition, unless otherwise specified.

In accordance with the present invention the bicyclic aromatic flavour compound is used for the manufacture of a composition for mast cell stabilization. The bicyclic aromatic flavour compound can be used in combination with an auxiliary mast cell stabilizer ingredient or it can be present as the sole or primary active ingredient. Where the bicyclic aromatic flavour compound is used as the sole or primary active antihistamine ingredient, however, the compositions herein are preferably essentially free of vasoconstrictor decongestant active materials, such as oxymetazoline.

The bicyclic aromatic flavour compound is selected from eucalyptol and camphor, and mixtures thereof. The highly preferred aromatic flavour compound for use in the present invention is camphor. The aromatic flavour compounds are preferably incorporated in the compositions herein at a level in the range of from about 0.005 % to about 0.2 %, more preferably from about 0.05 % to about 0.1 % by weight of final composition. The aromatic flavour compound additionally acts as a flavouring agent in the compositions.

The preferred auxiliary mast cell stabilizer ingredient in the compositions herein is sodium cromoglycate. The auxiliary mast cell stabilizer ingredient is preferably incorporated in the compositions at a level of from about 0.1 % to about 10 %, preferably from about 0.05 % to about 5.0 % by weight of composition.

The compositions herein are preferably provided in the form of an aqueous solution containing a solubilizing agent in order to solubilize the bicyclic aromatic flavour agent. The preferred solubilizer of the present invention is a 4-(1,1,3,3-tetramethylbutyl) phenol polymer with formaldehyde and oxirane. This is sold under the trade mark Tyloxapol^{(RTM)}. The solubilizer is generally present at a level of from about 0.1 % to about 2 %, preferably from about 0.5 % to about 1.0 % by weight of the compositions.

The compositions may additionally comprise an antihistamine selected from diphenhydramine hydrochloride, chloropheniramine, chloropheniramine maleate, doxylamine succinate, bromopheniramine maleate, terfenadine, cetirizine, astamizole, loratidine, azelastine, acrivastine and clemastine, preferably chloropheniramine. A topical steroidal agent can also be added to the composition, such as hydrocortisone, prednisone, prednisolone, triaminolone, triamcinolone acetonide, dexamethasone, bunedoside, flunisolide, betamethasone, beclomethasone dipropionate and beclomethasone, preferably beclomethasone. These additional materials can be generally added in a level from about 0.01 % to about 5 % by weight of total composition.

The pharmaceutical compositions can be complemented by various optional ingredients including one or more sequestering agents, preservatives, solvents and cationic surfactants.

Suitable sequestering agents for incorporation herein include polycarboxylates, amino polycarboxylates, polyphosphates, polyphosphonates and aminopolyphosphonates such as ethylenediaminetetra-acetic acid (EDTA), diethylenetriaminepenta-acetic acid, citric acid, gluconic acid, pyrophosphoric acid, etc. as well as their water-soluble salts, especially the alkali metal, ammonium and alkanol ammonium salts. Sequestering agents are generally employed in amounts in the range from about 0.001 % to about 0.2 %, preferably from about 0.01 % to about 0.1 % by weight of the final pharmaceutical composition.

The pharmaceutical compositions can additionally include preservatives. These can be water-soluble or solubilizable preservatives such as DMDM Hydanton^{(RTM)}, Germall^{(RTM)} 115, methyl, ethyl, propyl, and butyl esters of hydrobenzoic acid, benzoic acid, Euxyl^{(RTM)} K400, Bronopol^{(RTM)} (2-bromo-2-nitropropane-1,3-diol), sodium benzoate, chlorhexadine, benzalkonium chlorides and 2-phenoxyethanol, parabens, benzyl alcohols, chlorbutol, phenoxyethanol, Cetrimide, potassium sorbate and thiomersal. A preferred preservative for inclusion in the compositions herein is thiomersal. In general, amounts of from about 0.0005 % to about 0.05 % are suitable herein with amounts of from about 0.001 % to about 0.01 % being preferred.

Additionally, solvents can be added to the compositions herein. Suitable solvents encompass lower alkyl alcohols, diols and polyols containing from one to six carbon atoms, preferably from two to four carbon atoms, for example, ethanol, propylene glycol, isopropanol, butanol, glycerol, etc. The solvent can be present in an amount from about 0.1 % to about 50%, preferably from about 1 % to about 20 % by weight of the composition. The balance of the composition is water.

Cationic surfactants can be added to the compositions for their germicidal activity. Suitable cationic surfactants are generally quaternary ammonium compounds such as the alkylarylether dimethylbenzylammonium chlorides, for example, 2-[2-(p-octylcresoxy) ethoxy] ethyl dimethylbenzylammonium chloride. This is commercially available under the name methylbenzethonium chloride or Hyamine^{(RTM)} 10X. The corresponding p-octylphenoxy derivative of this compound is commercially available as benzethonium chloride, and is also suitable for the present purpose, as are the alkyldimethylbenzylammonium chlorides available under the name benzalkonium chloride. Other suitable cationic surfactants include alkyltrimethylammonium chlorides of which cetyltrimethylammonium chloride is commercially available under the trade name Cetab^{(RTM)}; alkyldimethylethylammonium halides; alkylpyridinium chloride, for example, cetylpyridinium chloride; alkylimidazolinium chloride such as alkylhydroxyethylimidazolinium chloride; alkyldimethyldichlorobenzylammonium chlorides; acylcolaminoformylinethyl pyridinium chlorides available under the name of Emulsept^{(RTM)}; and alkylarylmethylpyridinium chlorides such as polyalkylnaphthalene methylpyridinium chloride available under the trade name Emcol^{(RTM)}. The term "alkyl" in the aforementioned cationic surfactants refers to alkyl radicals containing from eight to eighteen carbon atoms, preferably from ten to sixteen carbon atoms, and mixtures thereof, this being the range in which quaternary ammonium compounds are considered to have good germicidal activity. These cationic surfactants can be present in a range from about 0.001 % to about 0.1 % by weight of composition.

The compositions herein can optionally contain one or more other known therapeutic agents, particularly those commonly utilized in cough/cold preparations, such as pharmaceutically-acceptable decongestants, analgesics and expectorants. Suitable decongestants include, for example, pseudoephedrine hydrochloride, pseudoephedrine, phenylephedrine hydrochloride, phenylephrine, ephedrine hydrochloride, phenylpropanolamine, ephedrine sulphate, oxymetazoline, xylometazoline, antazoline, naphazoline and tramazoline; analgesics include acetaminophen and ibuprofen; while suitable expectorants include glyceryl guaiacolate, terpin hydrate and ammonium chloride.

In preferred embodiments the compositions herein are preferably provided in the form of a preferably pump-actuated aerosol spray. Spray compositions herein preferably have a viscosity in the range from about 1 to about 100 cps, more preferably from about 1 to about 50 cps. Viscosity is measured using a Brookfield RVT, Spindle RV1 at 100 rpm and 25°C. The spray pump for use in the adminstering of unit dosages of pharmaceutical compositions herein is preferably of a finger actuated pump design. The compositions are applied intra-nasally in a unit dosage containing from about 0.01 mg to about 0.1 mg, preferably from about 0.02 mg to about 0.06 mg of camphor per unit dose and from about 1 mg to about 10 mg, preferably from 2 mg to 6 mg of auxiliary mast cell stabilizer per unit dose. In the preferred spray pump device there are approximately 1 to 3 sprays per unit dose.

### Mast Cell Stabilization Test

Peritoneal rat mast cells are collected from 250 to 300 g Wistar male rats by the following procedure. Following sacrifice, each rat is injected into the peritoneal cavity with 10 ml of ice cold HEPES-buffered salt solution (HBSS), pH 7.2, containing 5 U/ml heparin. The HBSS contains 137 mM of KCl, 10mM HEPES, 5mM D-glucose, 2.7 mM KCl, 0.4 mM NaH₂PO₄, 0.5 mM MgCl₂ and 1 % foetal calf serum (FCS). The abdomen is massaged gently for 2 to 3 minutes and then an incision is made in the abdomen, the buffer removed by a pipette, and the peritoneal cavity lavaged twice with ice-cold HBSS. The extract is then centrifuged at 200 rpm for 10 minutes at 20°C to collect the peritoneal mast cells. These cells are then suspended again in HBSS. A count of cells present is made using an aliquot of this suspension by staining with Kimura metachromatic stain. Typically, about 1.2 to 1.7x10⁶ mast cells are collected from each animal.

The rat mast cells are then activated using a Compound 48/80 in the presence of the test aromatic and antihistamine compounds and histamine-release measured as follows. 720 µl aliquots containing 9 x 10⁴ mast cells at 37°C are added to vials containing various combinations of aromatic flavour compounds. The concentrations of tyloxapol, menthol, camphor, and eucalyptol are 0.7 %, 0.025 %, 0.015 %, and 0.0075 % respectively. The solutions are incubated for about 10 minutes before 200 µl aliquots are added to tubes containing 25 µl of Compound 48/80, 25 µl of sodium cromoglycate and/or buffer, giving a concentration of Compound 48/80 and sodium cromoglycate in the tubes of 0.1 g/ml and 0.005 % respectively. The histamine release reactions in the tubes is terminated after 10 minutes (37°C) by adding 750 µl of ice-cold HBSS and centrifuging the resulting solution for 10 minutes at 2000 rpm at 4°C. The histamine released by stimulus and the total cell histamine content are respectively determined by assaying the supernatant liquid after acidification with 5 % trichloroacetic acid (TCA) and by disintegrating the cells in 5 % (TCA) followed by analysis by automated spectrofluoremetry.

### Results

### i) Induced Histamine Release

The results from pretreating the rat peritoneal mast cells with various combinations of aromatic compounds showed that at the concentrations used in the experiment, sodium cromoglycate, menthol, camphor and eucalyptol did not induce release of histamine, while tyloxapol did induce net histamine release (5.18 ± 1.08 %). (The net histamine release induced by compound 48/80 at the concentration used (0.1 µg/ml) was about three times the net histamine release induced by tyloxapol (15.07 ± 2.0 %)).

### ii) Histamine Release Inhibition

In the presence of 100 µM of sodium cromoglycate, Compound 48/80-induced histamine release was inhibited by 67 % and tyloxapol-induced histamine release by 152 %. In a series of five experiments, camphor at 0.015 % was found to inhibit histamine release by 45 % against Compound 48/80 and tyloxapol-induced histamine release. Eucalyptol at 0.0075 % gave a 9 % (non-significant) inhibitory effect. Menthol at 0.025 % was not effective.

### Example 1

Pharmaceutical compositions herein are prepared with the following formula:
0.015 % camphor
0.025 % menthol
0.0075 % eucalyptol
2 % sodium cromoglycate
0.002 % thiomersal (preservative)
0.05 % EDTA
0.7 % tyloxapol solubilizer
and water to 100 %.

### Example 2

A second composition is prepared with the following formula:
0.015 % camphor
0.002 % thiomersal
0.06 % EDTA
0.8 % tyloxapol solubilizer
and water to 100 %.

### Example 3

A third pharmaceutical composition is prepared having the following ingredients:
0.018 % camphor
2.3 % sodium cromoglycate
0.003 % thiomersal
0.06 % EDTA
0.7 % tyloxapol solubilizer
and water to 100 %

### Example 4

A fourth composition is prepared as follows:
0.008 % eucalyptol
0.002 % thiomersal
0.07 % EDTA
0.7 % tyloxapol solubilizer
and water to 100 %

The pharmaceutical compositions of the above Examples demonstrate effective mast cell stabilization and antihistamine activity.

## Claims

1. The use of a bicyclic aromatic flavour compound for the manufacture of a composition for the stabilization of mast cells, wherein the bicyclic aromatic flavour compound is selected from camphor, eucalyptol and mixtures thereof.

2. The use according to claim 1 wherein the composition comprises the bicyclic aromatic flavour compound as the sole or primary active ingredient.

3. The use according to claim 1 or 2 wherein the composition is essentially free of vasoconstrictor decongestant active materials.

4. The use according to any of claims 1 to 3 wherein the composition is in the form of an aqueous solution comprising the bicyclic aromatic flavour compound and a solubilizer therefor.

5. The use according to claim 4 wherein the aromatic flavour agent is camphor.

## Patentansprüche

1. Verwendung einer bicyclischen aromatischen Aromaverbindung zur Herstellung einer Zusammensetzung zur Stabilisierung von Mastzellen, worin die bicyclische aromatische Aromaverbindung unter Kampfer, Eucalyptol und Gemischen hievon ausgewählt wird.

2. Verwendung nach Anspruch 1, worin die Zusammensetzung die bicyclische aromatische Aromaverbindung als den einzigen oder hauptsächlichen wirksamen Bestandteil umfaßt.

3. Verwendung nach Anspruch 1 oder 2, worin die Zusammensetzung im wesentlichen frei von gefäßverengenden, abschwellend wirksamen Materialien ist.

4. Verwendung nach einem der Ansprüche 1 bis 3, worin die Zusammensetzung in Form einer wäßrigen Lösung vorliegt, die die bicyclische aromatische Aromaverbindung und einen Solubilisator hiefür umfaßt.

5. Verwendung nach Anspruch 4, worin das aromatische Aromamittel Kampfer ist.

## Revendications

1. Utilisation d'un composé aromatique bicyclique aromatisant pour la fabrication d'une composition pour la stabilisation des mastocytes, dans laquelle le composé aromatique bicyclique aromatisant est choisi parmi le camphre, l'eucalyptol, et leurs mélanges.

2. Utilisation selon la revendication 1, dans laquelle la composition comprend le composé aromatique bicyclique aromatisant comme seule ou principale substance active.

3. Utilisation selon la revendication 1 ou 2, dans laquelle la composition est essentiellement dépourvue de substances actives décongestionnantes vasoconstrictrices.

4. Utilisation selon l'une quelconque des revendications 1 à 3, dans laquelle la composition est sous forme d'une solution aqueuse comprenant le composé aromatique bicyclique aromatisant et un solubilisant de ce composé.

5. Utilisation selon la revendication 4, dans laquelle l'agent aromatique aromatisant est le camphre.
